# EUROPÄISCHE PATENTSCHRIFT

(11) **EP 0 606 548 B1**
(45) Veröffentlichungstag und Bekanntmachung des Hinweises auf die Patenterteilung: **07.02.1996**
(21) Anmeldenummer: 93117784.4
(22) Anmeldetag: 03.11.1993
(51) Int. Cl.: A61B 17/22

(54) **Extrakorporales Therapiegerät**
Apparatus for extracorporal therapy
Appareil de thérapie extracorporelle

(30) Priorität: 14.01.1993 DE 4300740
(43) Veröffentlichungstag der Anmeldung: 20.07.1994
(73) Patentinhaber: Richard Wolf GmbH, D-75438 Knittlingen (DE)
(72) Erfinder: Bauer, Edgar, D-76703 Kraichtal (DE); Krauss, Werner, D-75434 Knittlingen (DE); Stettner, Dietmar, D-75417 Mühlacker (DE)
(74) Vertreter: Vollmann, Heiko, Dipl.-Ing.

(56) Entgegenhaltungen:
- EP-A- 0 396 866
- EP-A- 0 402 584
- EP-A- 0 538 659
- DE-A- 3 916 093

## Beschreibung

Die Erfindung betrifft ein extrakorporales Therapiegerät mit den im Oberbegriff des Anspruchs 1 aufgeführten Merkmalen.

Derartige Therapiegeräte werden beispielsweise bei der Zertrümmerung von Konkrementen oder der Behandlung von Gewebe (Tumorbehandlung) eingesetzt. Sie arbeiten in der Regel mit Hochenergie-Ultraschall, sei es in Form von Stoßwellen oder im Dauer- oder Burstbetrieb.

Ein gattungsgemäßes Gerät ist aus DE 39 16 093 A1 bekannt. Der dort beschriebene Lithotriptor weist einen kalottenförmigen Wandler mit darin integrierter Ultraschallortungseinrichtung und Röntgenortungseinrichtung auf. Wandler und Ortungseinrichtungen sind fest miteinander verbunden und können in bezug auf die Patientenauflage um eine konfokale Achse geschwenkt werden. Da beim Schwenken des Wandlers mit seinen Ortungseinrichtungen der Fokus unverändert bleibt, können ohne Lageveränderung des Patienten die Richtung der Schallwellen geändert werden, was von großem Vorteil ist. So können beispielsweise Hindernisse im Schallweg umgangen werden. Auch für die Ortung bietet dies besondere Vorteile, da der Fokusbereich innerhalb des Patientenkörpers aus unterschiedlichen Richtungen und Blickwinkeln betrachtet werden kann, wodurch exakte Daten über die räumliche Erstreckung etwaiger Konkremente oder zu behandelnder Gewebebereiche erhalten werden können.

Als nachteilig bei derartigen mit Röntgen- und Ultraschallortung ausgestatteten Therapiegeräten wird der vergleichsweise hohe Anschaffungspreis empfunden. Bei der immer vielseitiger werdenden Anwendung sowohl der Therapiegeräte als auch der Ortungseinrichtungen gibt es Bestrebungen, diese zumindest zeitweise voneinander zu trennen, um sie unabhängig voneinander nutzen zu können und somit die Wirtschaftlichkeit zu erhöhen.

So reicht bei zahlreichen Anwendungen der vorerwähnten Lithotriptoren häufig die Ultraschallortungseinrichtung aus. Es sind daher schon Therapiegeräte bekannt, bei denen für den Fall einer gewünschten Röntgenortung eine gesonderte Röntgenortungseinrichtung an das Therapiegerät herangestellt werden muß. Hier kann dann ein handelsüblicher Röntgen-C-Bogen Verwendung finden. Voraussetzung ist jedoch, daß das Therapiegerät so ausgebildet ist, daß noch genügend Raum zum Einschieben dieses C-Bogens verbleibt.

Eine solche Anwendung von extrakorporalem Therapiegerät in Verbindung mit einer externen Röntgenortungseinrichtung ist beispielhaft in EP 0 402 584 A1 beschrieben. Dort geht es im wesentlichen um das sich regelmäßig bei solchen Anordnungen stellende Problem der räumlichen Zuordnung zwischen Röntgenortungseinrichtung und dem Fokus des elektroakustischen Wandlers. Dieses Problem wird durch eine Art Zieleinrichtung gelöst, die am Wandler angebracht wird und anhand der der Röntgenbogen in zwei Stellungen auf den Fokus ausgerichtet wird. Die hierzu erforderlichen Justierarbeiten sind nicht nur zeitaufwendig, sondern belasten den Patienten auch wegen der dabei auftretenden Röntgenstrahlung. Ein weiterer Nachteil ist darin zu sehen, daß immer dann, wenn die Stellung des Wandlers in bezug auf den Patienten geändert wird, wenn beispielsweise bestimmte Körperbereiche abgeschattet und daher von einer Seite nicht zugänglich sind, stets die Röntgenortungseinrichtung neu ausgerichtet werden muß.

Aus der nachveröffentlichten EP 0 538 659 A2 ist es bereits bekannt, eine Röntgenortungseinrichtung in Form eines externen Röntgen-C-Bogens im Bedarfsfalle an ein extrakorporales Therapiegerät derart anzukoppeln, daß es zusammen mit dem Therapiegerät um den Fokus des Wandlers schwenkbar ist. Auch dort ist bereits eine Zieleinrichtung vorgesehen, mit der die Röntgenortungseinrichtung in bezug auf den Wandlerfokus ausrichtbar ist. Die dort beschriebene Zieleinrichtung ist im Strahlengang zwischen Röntgenquelle und Röntgenbildempfangsvorrichtung in Form von zwei in Überdeckung zu bringende Zielmarken vorgesehen bzw. in diesen Strahlengang einschwenkbar, und zwar außerhalb des Fokus des Wandlers.

Ausgehend von dem einleitend genannten Stand der Technik nach DE 39 16 093 A1 liegt der vorliegenden Erfindung die Aufgabe zugrunde, ein gattungsgemäßes Therapiegerät so auszubilden, daß einerseits die Röntgenortungseinrichtung vom Therapiegerät getrennt werden kann, andererseits diese aber schnell und einfach unter Vermeidung der vorgenannten Nachteile mit diesem verbunden und auf dieses ausgerichtet werden kann.

Erfindungsgemäß wird dies dadurch erreicht, daß die Röntgenortungseinrichtung als gesondertes Gerät, beispielsweise als Röntgen-C-Bogen ausgebildet und lediglich über eine Kupplungseinrichtung lösbar mit dem Therapiegerät verbunden wird. Um eine schnelle und genaue Ausrichtung der Röntgenortungseinrichtung in bezug auf das Therapiegerät, insbesondere den Wandlerfokus zu gewährleisten, sieht die Erfindung eine in den Fokus des Wandlers einbringbare Zielmarke für die Röntgenortungseinrichtung am Therapiegerät vor. Da Wandler und Röntgenortungseinrichtung um dieselbe Achse schwenkbar angeordnet sind, genügt die Ausrichtung auf diese Zielmarke im Fokus des Wandlers.

Die erfindungsgemäße Ausbildung hat den Vorteil, daß die bei Therapiegeräten mit fest zugeordneten Röntgenortungseinrichtungen, wie sie beispielsweise aus DE 39 16 093 A1 bekannt sind, bekannten Vorteile im wesentlichen erhalten bleiben - hier ist insbesondere die Schwenkbewegbarkeit des Wandlers hervorzuheben -, jedoch die bekannten Nachteile, die mit externen Röntgenortungseinrichtungen bekannter Art einhergehen, im wesentlichen vermieden werden können. Die zum Anschluß der Röntgenortungseinrichtung an das Therapiegerät erforderliche Kupplung kann, soweit es die Röntgenortungseinrichtung betrifft, ohne weiteres nachgerüstet werden, so daß die handelsüblichen Röntgen-C-Bögen Verwendung finden können. Diese sind ohnehin verfahrbar und meist auch in der Höhe ihrer Schwenkachse einstellbar, so daß die Schwenkachse der Röntgenortungseinrichtung und des Wandlers zumindest im wesentlichen durch manuelles Ausrichten in Übereinstimmung gebracht werden können.

Es stellt jedoch kein Problem dar, wenn diese Achsen nicht miteinander fluchten sondern geringfügig auseinanderfallen. Gemäß der Erfindung sind Zusatzeinrichtungen vorgesehen, mit denen in einfacher und sehr praktikabler Weise eine exakte Zuordnung der Röntgenortungseinrichtung zum Fokus des Wandlers gewährleistet ist, und das über den gesamten Schwenkbereich des Wandlers. Nach dem Einrichten und Ankuppeln der Röntgenortungseinrichtung bleibt diese Zuordnung so lange erhalten, bis die Geräte wieder voneinander getrennt werden.

In einer Weiterbildung der Erfindung bestehen diese Zusatzeinrichtungen zum einen aus der Zielmarke für die Röntgenortungseinrichtung, die in den Fokus des Wandlers einbringbar ist, und zum anderen aus einer der Röntgenortungseinrichtung zugeordneten Steuerung, die in Verbindung mit einem dem Wandler zugeordneten Positionsgeber die jeweilige Wandlerstellung und damit auch Schwenkstellung der Röntgenortungseinrichtung erkennt und damit die Fokuslage in bezug auf die Röntgenortungseinrichtung anhand einer vorher ermittelten Wegkurve zuordnet. Zur Ermittlung dieser Wegkurve ist es lediglich erforderlich, die Lage des Fokus mit Hilfe der Zielmarke in zwei Schwenkstellungen, vorzugsweise den Endpositionen, mit Hilfe der Röntgenortungseinrichtung zu ermitteln, wonach die Steuereinrichtung diese Kurve bzw. die entsprechenden Werte ermittelt und bei der Darstellung der elektronischen Zielmarke auf den Monitor berücksichtigt.

Zweckmäßigerweise ist die Zielmarke für die Röntgenortungseinrichtung bereits am Therapiegerät, insbesondere dem Wandler vorgesehen, so daß diese bei Bedarf nur noch in die Fokuslage gebracht werden muß. Dies kann zweckmäßigerweise dadurch erfolgen, daß innerhalb des Wandlers oder nahe am Wandler eine ein- und ausfahrbare Teleskopeinrichtung vorgesehen ist, an deren Ende diese Zielmarke sitzt, so daß bei ausgefahrenem Teleskop die Zielmarke im Fokus des Wandlers liegt und bei eingefahrenem Teleskop weder Therapie noch Ortung behindert.

Vorteilhaft kann dies auch durch eine Anbringung der Zielmarke am patientenseitigen Ende des Ultraschallscanners vorgesehen sein. Eine zweckmäßige Vorrichtung zum Aus- und Einschwenken dieser Zielmarke am Ultraschallscanner ist weiter unten anhand eines Ausführungsbeispiels beschrieben.

Die Zielmarke kann jedoch auch auf der die geräteseitige Vorlaufstrecke zum Patienten hin abschließenden Kuppelmembran angebracht werden, indem dort eine entsprechende röntgenpositive Marke aufgeklebt wird. Um auch sicherzustellen, daß diese Marke tatsächlich im Fokus des Wandlers angeordnet ist, wird zweckmäßigerweise eine optische Einrichtung vorgesehen, die beispielsweise aus zwei innerhalb des Wandlers angeordneten Lasern besteht, deren Strahlen sich im Fokus des Wandlers schneiden. Wenn die beiden sich auf der Membran bildenden Leuchtflecke durch Verschiebung der Membran in Überdeckung gebracht sind, befindet sich die entsprechende Stelle der Membran im Fokus des Wandlers. Es kann dann an dieser Stelle eine röntgenpositive Zielmarke aufgeklebt werden.

Die bereits angesprochenen handelsüblichen Röntgen-C-Bögen weisen an einem Ende des C eine Röntgenquelle und am anderen Ende einen Röntgenbildverstärker auf, dessen Signal einer Bildwiedergabeeinrichtung, in der Regel einem Monitor, zugeführt wird. Auf diesem Monitor ist üblicherweise eine Zielmarke darstellbar, die mit dem Zentralstrahl der Röntgenquelle übereinstimmt. Bei Anordnungen nach dem Stand der Technik ist es stets so, daß dieser Zentralstrahl und damit die im Monitor festgelegte Zielmarke mit dem Wandlerfokus in Übereinstimmung gebracht werden muß. Eine solche milimetergenaue Positionierung ist sehr zeitaufwendig. Die erfindungsgemäße Lösung sieht hingegen nur eine manuelle Grobausrichtung vor und weist in einer vorteilhaften Weiterbildung die Möglichkeit auf, die elektronische Zielmarke auf die tatsächlich abgebildete Zielmarke nachzuführen. Hiermit ist es also ohne exakte Ausrichtung der Röntgenortungseinrichtung möglich, den Fokus des Wandlers auf dem Monitor darzustellen, auch wenn die tatsächliche Zielmarke sich nicht im Bereich des Zentralstrahls befindet.

Um dies auch mit der erforderlichen Genauigkeit in jeder Schwenkstellung des Wandlers und der Röntgenortungseinrichtung zu gewährleisten, ist gemäß der Weiterbildung der Erfindung eine Steuereinrichtung vorgesehen, welche die elektronische Zielmarke entsprechend der jeweiligen Schwenkstellung des Wandlers nachführt, und zwar auf einer vorher anhand der tatsächlichen Fokuslage in den Schwenkendstellungen ermittelten Bahn.

Um an der Röntgenortungseinrichtung möglichst wenig bauliche Änderungen vornehmen zu müssen, ist es zweckmäßig, nicht die Schwenkstellung der Röntgenortungseinrichtung, sondern die des Wandlers innerhalb des Therapiegerätes zu erfassen und der Steuereinrichtung zuzuführen. Eine solche Erfassung kann durch einen am Wandler vorgesehenen Wegaufnehmer erfolgen. Wegaufnehmer im Sinne der Erfindung ist ein Sensor, der die Stellung des Wandlers erfaßt, sei es durch Wegmessung, durch Winkelmessung oder auf andere Weise.

Von Vorteil ist es, wenn die Kupplungseinrichtung, die die Röntgenortungseinrichtung am Therapiegerät festlegt therapiegeräteseitig nahe dem Wandler und auf Seiten der Röntgenortungseinrichtung am Traggestell, und zwar in der Nähe der Röntgenquelle angeordnet ist. Hierdurch wird eine Verbindung über kurze Hebel erreicht und zudem eine recht genaue Anordnung zwischen Röntgenquelle und Wandler gewährleistet. Insbesondere bei einem kalottenförmig ausgebildeten und aus piezoelektrischen Elementen aufgebauten Wandler ist es zweckmäßig, wenn innerhalb der Wandlerkalotte ein Fenster für die Röntgenquelle vorgesehen ist.

Neben der vorrichtungsmäßigen Ausbildung gibt die Erfindung auch ein einfach und schnell durchzuführendes Verfahren zum Einrichten einer externen Röntgenortungseinrichtung an einem solchen Therapiegerät an, mit dem eine Zuordnung zwischen Wandlerfokus und Röntgenortungseinrichtung entsprechend der jeweiligen Schwenkstellung erfolgt. Nach dem Ankuppeln der Röntgenortungseinrichtung an den Wandler des Therapiegerätes, also nach der mechanischen Verbindung der Aggregate, wird der Wandler zusammen mit der Röntgenortungseinrichtung in eine Endstellung geschwenkt. Dann wird die Zielmarke für die Röntgenortung, also eine röntgenpositive Marke, im Fokus des Wandlers angebracht (dies kann natürlich auch in beliebiger Zeit vorher erfolgen). Mittels der Röntgenortungseinrichtung wird die Zielmarke auf der Bildwiedergabeeinrichtung abgebildet und die Lage dieser Zielmarke in einer Steuereinrichtung gespeichert. Sodann wird der Wandler zusammen mit der Röntgenortungseinrichtung in seine andere Endstellung geschwenkt und wiederum die Lage der abgebildeten Zielmarke gespeichert. Nachdem die Zielmarke aus dem Fokus entfernt worden ist, ist das Einrichten beendet. Die Steuereinrichtung errechnet nun eine Bahn, welche die Lage des Wandlerfokus in Abhängigkeit der Schwenkstellung des Wandlers angibt. Die in der Bildwiedergabeeinrichtung vorgesehene elektronische Zielmarke wird anhand dieser Bahnparameter nachgeführt, so daß die elektronische Zielmarke der Röntgenortungseinrichtung stets mit hoher Genauigkeit der tatsächlichen Fokuslage des Wandlers entspricht.

Die Erfindung ist nachfolgend anhand von in den Figuren dargestellten Ausführungsbeispielen erläutert. Es zeigen:
- Figur 1: in schematischer Darstellung ein Therapiegerät mit angekuppelter Röntgenortungseinrichtung in Seitenansicht,
- Figur 2: die Einzelheit II in Figur 1 in vergrößerter Teilschnittdarstellung,
- Figur 3: eine Ansicht des Kupplungsbereichs zwischen Therapiegerät und Röntgenortungseinrichtung von der Rückseite der Figur 2,
- Figur 4: eine Ansicht in Richtung des Pfeils IV in Figur 3,
- Figur 5: die Anordnung einer Zielmarke am Ultraschallscanner,
- Figur 6: in vergrößerter Darstellung das patientenseitige Ende des Ultraschallscanners mit Zielmarke,
- Figur 7: eine Draufsicht in Richtung des Pfeils VII in Figur 6 mit seitlich weggeschwenkter Zielmarke,
- Figur 8: eine andere Anbringung einer Zielmarke innerhalb des Wandlers,
- Figur 9: eine weitere Ausführung der Anbringung einer Zielmarke,
- Figur 10: eine alternative Einrichtung zur Anordnung einer Zielmarke am Wandler,
- Figur 11: eine Draufsicht auf die Koppelmembran des anhand von Figur 10 dargestellten Wandlers,
- Figur 12: die elektronische Kopplung zwischen Wandler und Röntgenortungseinrichtung im Blockschaltbild und
- Figur 13: die Verfahrensschritte beim Einrichten anhand von Monitordarstellungen.

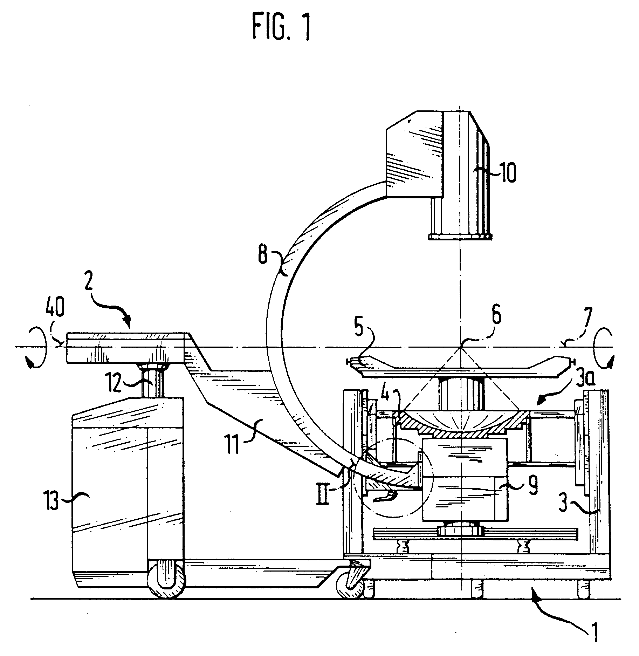

Die Anordnung nach Figur 1 zeigt ein extrakorporales Therapiegerät 1 mit angekuppelter Röntgenortungseinrichtung 2. Das Therapiegerät 1 besteht im wesentlichen aus einem Tragrahmen 3, in dem ein elektroakustischer Wandler 4 schwenkbar gelagert ist und an dem eine Patientenauflage 5 verstellbar angebracht ist. Die üblicherweise erforderlichen Nebenaggregate, wie Steuerelektronik, Stelleinrichtungen usw. sind nicht dargestellt. Der Wandler 4 besteht aus einer Vielzahl von kalottenförmig angeordneten piezoelektrischen Elementen, sein Fokus ist mit 6 bezeichnet. Der Wandler 4 ist um die Achse 7, in der der Fokus 6 liegt, schwenkbar auf einem Schwenktisch 3a innerhalb des Tragrahmens 3 gelagert. Er kann aus der dargestellten, zur Patientenauflage 5 senkrechten Position jeweils um 15° zu beiden Seiten ausgeschwenkt werden. Um einen auf der Patientenauflage 5 befindlichen Patienten zu positionieren, kann die Patientenauflage 5 in allen drei Raumkoordinaten zum Tragrahmen 3 und zum Wandler 4 positioniert werden.

Die Röntgenortungseinrichtung 2 ist als handelsüblicher Röntgen-C-Bogen ausgebildet. Der eigentliche C-Bogen 8 ist Teil des Traggestelles. An einem Ende dieses Bogens 8 ist eine Röntgenquelle 9 angeordnet, an der gegenüberliegenden Seite ein Röntgenbildverstärker 10. Der C-Bogen 8 ist als Profil ausgebildet und in Richtung seines Bogens an einem Arm 11 geführt, der seinerseits über eine (nicht im einzelnen dargestellte) Hubeinrichtung 12 mit dem Fahrgestellt 13 verbunden ist. Der Arm 11 ist so an der Hubeinrichtung 12 gelagert, daß der gesamte C-Bogen mit dem Arm 11 um die Achse 40 schwenkbar ist, die nicht notwendigerweise exakt mit der Achse 7 zusammenfallen muß.

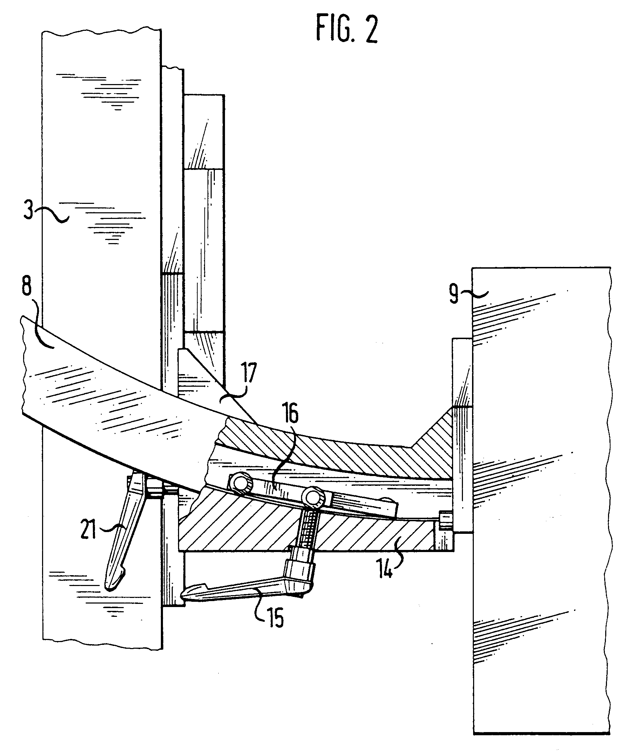

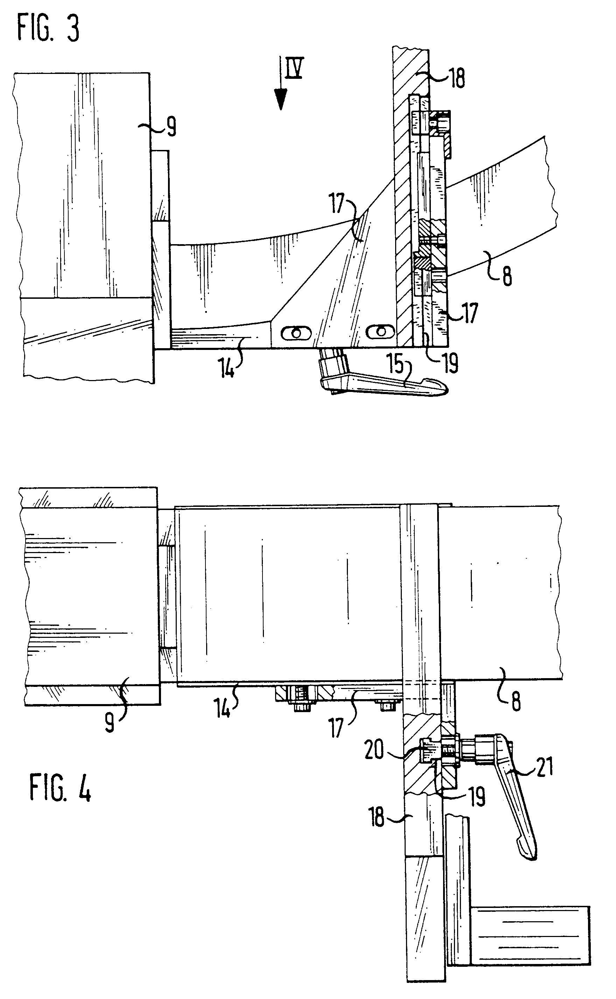

Zum Anschluß der Röntgenortungseinrichtung 2 an das Therapiegerät 1 wird zunächst einmal der C-Bogen 8 mittels der Hubeinrichtung 12 so ausgerichtet, daß die Achse 40 etwa mit der Achse 7 fluchtet. Dann wird der C-Bogen mit seinem unteren, die Röntgenquelle 9 tragenden Ende in den Tragrahmen 3 des Therapiegerätes 1 soweit eingeschoben, bis die Röntgenquelle 9 mit dem im Wandler 4 vorgesehenen (nicht dargestellten) Röntgenfenster in Überdeckung ist. In dieser Stellung ist dann die anhand der Figuren 2 bis 4 im einzelnen dargestellte Kupplung anzuschließen, die eine feste mechanische Verbindung zwischen den Geräten schafft.

Die Kupplung besteht aus einem C-bogenseitigem Teil und einem geräteseitigen Teil. C-bogenseitig ist mittels der ohnehin im C-Bogen 8 vorhandenen nach außen hin offenen Führungsnut des C-Bogens, nahe der Röntgenquelle 9 ein Kupplungskörper 14 festgelegt. Dieser Kupplungskörper 14 kann über einen Hebel 15, gegenüber einer innerhalb der Nut laufenden Spannplatte 16 festgelegt werden. Der Kupplungskörper 14 ist im wesentlichen quaderförmig, jedoch im Bereich des C-Bogens 8 an diesen angepaßt, so daß eine großflächige sichere Anlage gewährleistet ist. An den Kupplungskörper 14 schließt seitlich eine Winkelplatte 17 an, die fest mit dem Kupplungskörper 14 verschraubt ist. Ein Schenkel dieser Winkelplatte 17 kommt zur Anlage an einer Flachseite des Schwenktisches 3a, und zwar an einer Spannplatte 18. Diese Spannplatte 18 bildet den geräteseitigen Teil der Kupplung. Sie weist eine im Querschnitt T-förmige Nut 19 auf, in die ein den entsprechenden Schenkel der Winkelplatte 17 durchsetzender Spannbolzen 20 greift, der mittels eines außen an der Winkelplatte angreifenden Hebels 21 spannbar ist, mit dem die Winkelplatte 17 fest mit der Spannplatte 18 verbunden werden kann. Hier erfolgt also der eigentliche Kupplungsvorgang zwischen Röntgenortungseinrichtung 2 und Schwenktisch 3a im Therapiegerät 1. Aufgrund der großflächigen Anlageflächen der Kupplung können die hier auftretenden Kräfte sicher übertragen werden, insbesondere ist gewährleistet, daß der C-Bogen 8 den Schwenkbewegungen des Wandlers 4 folgt.

Da der C-bogenseitige Teil der Kupplung unter Ausnutzung des vorhandenen Profils festgelegt ist, brauchen am C-Bogen selbst keine baulichen Veränderungen vorgenommen zu werden. Dieser Teil kann ggf. vor Ort schnell und einfach am C-Bogen 8 montiert werden.

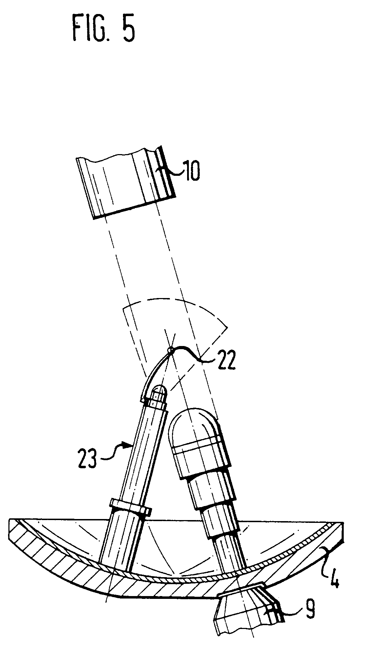

Wie bereits eingangs beschrieben, erfolgt mit dieser mechanischen Ankupplung zwischen Röntgenortungseinrichtung 2 und Therapiegerät 1 lediglich eine Grobausrichtung, die exakte Einrichtung erfolgt durch elektronische Nachführung der Zielmarke auf dem Monitor der Röntgenortungseinrichtung. Für diese Einrichtung ist die Anordnung einer röntgenpositiven Zielmarke 22 im Fokus 6 erforderlich, die dann mittels der Röntgenortungseinrichtung 2 darstellbar ist. Diese Zielmarke 22, z.B. in Form einer Bleikugel, wie sie in den Figuren 5 bis 9 dargestellt ist, kann auf verschiedene Weise angebracht sein.

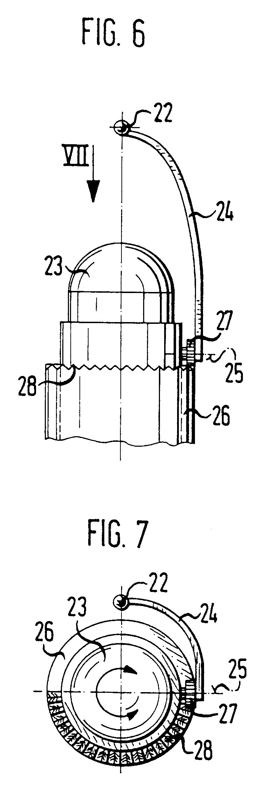

Anhand der Figuren 5 bis 7 ist eine Ausführung beschrieben, bei der diese Zielmarke am patientenseitigen Ende eines Ultraschallscanners 23 ausschwenkbar angeordnet ist. Die Zielmarke 22 ist über einen Arm 24 gehalten, der an seinem scannerseitigen Ende mit einer quer zur Längsachse des Scanners verlaufenden Achse 25 derart schwenkbar gelagert ist, daß die Zielmarke, wie dargestellt, in den Fokus einschwenkbar oder aber an den Scanner 23 anlegbar ist. Die Verschwenkung erfolgt über eine Drehung des Scanners 23 innerhalb eines feststehenden, diesen umgebenden Rohres 26. Auf der Achse 25 Sitzt ein fest mit dieser verbundenes stirnverzahntes Rad 27, das mit einer entsprechenden auf der freien Stirnseite des Rohres 26 angebrachten Verzahnung 28 kämmt. Die Verzahnung 28 erstreckt sich, wie in Figur 7 sichtbar, nur über einen Teil der Stirnseite, und zwar über einen Bogen von etwa 180°. Durch Drehen des Scanners 23 innerhalb des Rohrs 26 wird das Zahnrad 27 gedreht, wodurch der Arm 24 mit der Zielmarke 22 aus- bzw. einschwenkt, je nach Drehrichtung. Die den Fokus repräsentierende Stellung ist durch entsprechende (nicht dargestellte) Anschläge bestimmt.

Um während der Ultraschallortung in unterschiedlichen Ebenen scannen zu können, ist es üblich, den Ultraschallscanner 23 drehbar anzuordnen. Damit bei diesen während der Ortung üblichen Drehbewegungen die Zielmarke 22 nicht unbeabsichtigt ausgeschwenkt wird, ist ein Teil der Stirnseite des Rohres 26 unverzahnt, so daß erst dann, wenn der Scanner 23 über 180° zu beiden Seiten hinausgedreht wird, das Einschwenken der Zielmarke 22 erfolgt.

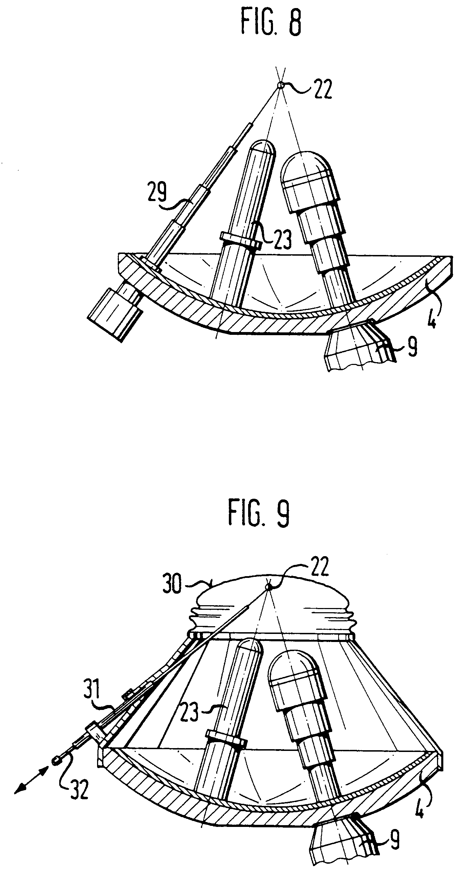

Eine alternative Vorrichtung zur Anbringung der Zielmarke 22 ist in Figur 8 dargestellt. Dort ist innerhalb der Kalotte des Wandlers 4 eine Teleskopeinrichtung 29 vorgesehen, mit der die Zielmarke 22 über das ausfahrbare Teleskop 29 im Fokus des Wandlers positioniert werden kann. Das Teleskop 29 kann mechanisch, pneumatisch oder hydraulisch betätigbar sein.

Bei der Ausführung nach Figur 9, in der der Wandler 4 mit seiner durch eine Membran 30 abgeschlossenen und flüssigkeitsgefüllten Vorlaufstrecke dargestellt ist, ist die Zielmarke 22 über ein seitlich des Wandlers angeordnetes Führungsrohr 31 an einer Stange 32 im Fokus 6 des Wandlers positionierbar. Das Führungsrohr 31 ist dicht in die Vorlaufstrecke eingegliedert, die Stange 32 ist verschiebbar und dicht innerhalb des Führungsrohrs 31 gelagert. Auch hier ist ein Anschlag vorgesehen, um die Fokusposition schnell und sicher zu erreichen. Im übrigen kann die Fokuspositon der Zielmarke 22 zusätzlich über den Ultraschallscanner 23 kontrolliert werden.

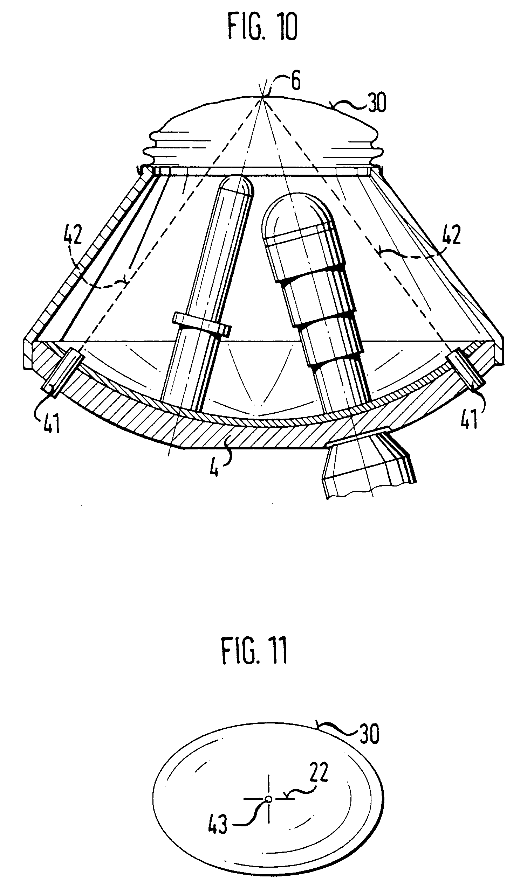

Anhand der Figuren 10 und 11 ist eine alternative Einrichtung zur Anbringung einer röntgenpositiven Zielmarke 22 dargestellt. Innerhalb des Wandlers 4 sind etwa diametral gegenüber angeordnet zwei HE-NE-Laser 41 vorgesehen, deren Lichtstrahlenbündel 42 sich im Fokus 6 des Wandlers 4 schneiden. Zum Anbringen der Zielmarke 22 wird nun zunächst die Koppelmembran 30 so lange in ihrem Abstand zum Wandler 4 verstellt, bis die durch die Lichtstrahlenbündel 42 auf der Membran 30 erzeugten Lichtflecken 43 in Übereinstimmung gebracht sind, wie dies in Figur 11 dargestellt ist. Dann wird an die Stelle der Lichtflecken 43 eine röntgenpositive Zielmarke 22, beispielsweise in Form eines Metallkreuzes auf der Membran aufgeklebt. Hierdurch ist die exakte Positionierung der Zielmarke 22 im Fokus 6 des Wandlers 4 sichergestellt.

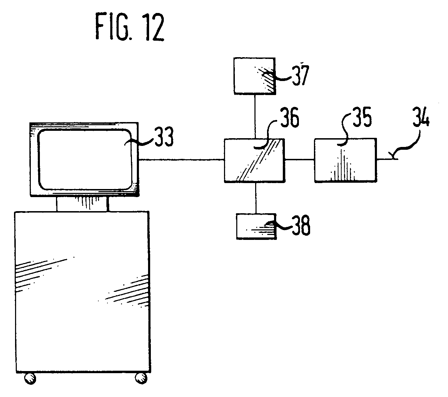

Anhand von Figur 12 ist die vorbeschriebene elektronische Korrektur verdeutlicht, die sicherstellt, daß die auf dem Monitor 33 dargestellte Zielmarke stets dem Fokus 6 des Wandlers entspricht. Das Videoausgangssignal des Röntgenbildverstärkers 10 ist in Figur 12 mit 34 bezeichnet. Dieses Signal durchläuft dann einen Zielmarkengenerator 35, der eine elektronische Zielmarke an der Stelle des Monitorbildes generiert. Dem Zielmarkengenerator 35 nachgeschaltet ist eine Steuereinrichtung 36, die über einen am Wandler angeordneten Wegaufnehmer 37 ein Signal über die Schwenkstellung des Wandlers erhält und über einen Speicher 38 Datenzugriff hat auf die tatsächlichen Koordinaten der abgebildeten Zielmarke 22 in den Endstellungen des Wandlers 4. Die Steuereinrichtung 36 errechnet anhand der im Speicher 38 abgelegten Koordinaten über die tatsächliche Fokuslage eine Bahn K, auf welcher der Fokus 6 beim Schwenken des Wandlers 4 um die Achse 7 bzw. beim Mitschwenken des Röntgen-C-Bogens 8 um die Achse 40 verfährt. Die sich daraus ergebenden jeweiligen Abweichungen werden ermittelt, die elektronische, vom Generator 35 erzeugte Zielmarke E wird nachgeführt und im Monitor 33 auf der dem Fokus 6 entsprechenden, errechneten Stelle dargestellt. Die im Speicher 38 festgelegten Werte werden beim Einrichten, wie eingangs beschrieben, ermittelt.

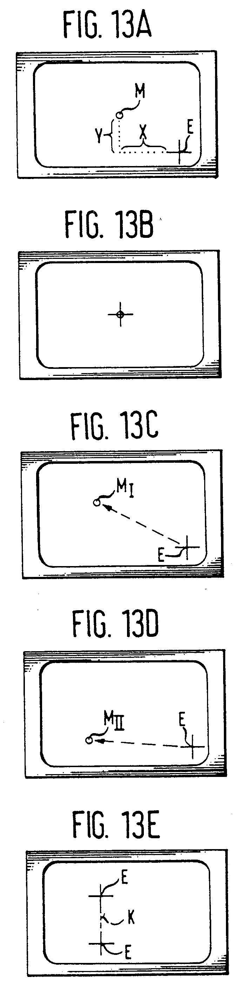

Anhand der Figur 13 wird der Einrichtvorgang nachfolgend erläutert:
Anhand der Figuren 13A und 13B ist zunächst einmal das Prinzip des Nachführens der elektronischen Marke auf die abgebildete Zielmarke erläutert. Die abgebildete Zielmarke 22 ist mit M gekennzeichnet, die elektronische Marke mit E. Um diese Marken in Überdeckung zu bringen, wie dies in Figur 13B dargestellt ist, sind die Koordinaten X und Y einzugeben. Eine solche Eingabe kann über Taster oder auch mit Hilfe eines optischen Zeigegerätes erfolgen. Im Speicher 38 werden die Koordinaten X und Y in den beiden Extremstellungen des Wandlers gespeichert. Diese Vorgänge sind beispielhaft anhand der Figuren 13C und 13D dargestellt. Es ist dabei deutlich sichtbar, daß die tatsächlichen Abbildungen der Zielmarke 22 in den beiden Schwenkstellungen I und II unterschiedliche Positionen aufweisen. Diese Abweichungen kommen beispielsweise dadurch zustande, daß die Achsen 40 und 7 nicht exakt miteinander fluchten. Nachdem in beiden Schwenkstellungen die elektronische Zielmarke E mit der tatsächlich abgebildeten Zielmarke M in Übereinstimmung gebracht und die entsprechenden Verschiebungskoordinaten gespeichert worden sind, wird innerhalb der Steuereinrichtung 36 eine Bahn K errechnet, welche die Position des Fokus in Abhängigkeit der Schwenkbewegung in bezug auf die Röntgenortungseinrichtung repräsentiert. Anhand dieser Bahn K wird entsprechend der jeweiligen Schwenkstellung bei der späteren Ortung die Zielmarke E auf dem Monitor 33 dargestellt.

## Patentansprüche

1. Extrakorporales Therapiegerät (1) mit einem elektroakustischen Wandler (4) zur Erzeugung von fokussierten Schallwellen, mit einer gegenüber dem Wandler (4) zum Zwecke der Positionierung eines Patienten verfahrbaren Patientenauflage (5) und mit einer Röntgenortungseinrichtung (2) - im wesentlichen bestehend aus einer Röntgenquelle (9), einer Röntgenbildempfangsvorrichtung (10) und einem diese verbindenden Traggestell (8), wobei der Wandler (4) zusammen mit der Röntgenortungseinrichtung (2) in Bezug auf die Patientenauflage (5) um den Fokus (6) des Wandlers derart schwenkbar angeordnet ist, daß die Röntgenortungseinrichtung (2) der Schenkbewegung des Wandlers (4) folgt, dadurch gekennzeichnet, daß die Röntgenortungseinrichtung (2) als gesondertes Gerät ausgebildet und über eine Kupplungseinrichtung lösbar mit dem Therapiegerät verbundene ist und daß eine in den Fokus (6) des Wandlers (4) einbringbare röntgenpositive Zielmarke (22) für die Röntgenortungseinrichtung (2) am Therapiegerät (1) vorgesehen ist.

2. Extrakorporales Therapiegerät nach Anspruch 1, dadurch gekennzeichnet, daß die Zielmarke (22) am Ende einer wandlerseitig angeordneten ein- und ausfahrbaren Teleskopeinrichtung (29) sitzt.

3. Extrakorporales Therapiegerät nach Anspruch 1, dadurch gekennzeichnet, daß die Zielmarke an einer an den Patienten anlegbaren Koppelmembran (30) des Wandlers (4) anbringbar ist.

4. Extrakorporales Therapiegerät nach Anspruch 3, dadurch gekennzeichnet, daß am oder innerhalb des Wandlers (4) zwei Lichtquellen (41), vorzugsweise Laser, angeordnet sind, deren Strahlen (42) sich im Fokus (6) des Wandlers (4) schneiden.

5. Extrakorporales Therapiegerät nach einem der vorhergehenden Ansprüche, dadurch gekennzeichnet, daß am Wandler (4) ein Ultraschallscanner (23) angeordnet ist und daß die Zielmarke (22) nahe dem patientenseitigen Ende des Ultraschallscanners (23) ausschwenkbar angeordnet ist.

6. Extrakorporales Therapiegerät nach einem der vorhergehenden Ansprüche, dadurch gekennzeichnet, daß die Röntgenbildempfangsvorrichtung (10) eine elektronische Bildwiedergabeeinrichtung (33) aufweist, auf der eine elektronische Zielmarke (E) darstellbar ist, und daß die elektronische Zielmarke (E) auf die tatsächlich abgebildete Zielmarke (M) nachführbar ist.

7. Extrakorporales Therapiegerät nach einem der vorhergehenden Ansprüche, dadurch gekennzeichnet, daß der Röntgenortungseinrichtung (2) eine elektronische Steuereinrichtung (36) zugeordnet ist, welche die elektronische Zielmarke (E) beim gemeinsamen Schwenken von Wandler (4) und Röntgenortungseinrichtung (2) auf einer vorher ermittelten Bahn (K) nachführt, wozu die jeweilige Schwenkstellung des Wandlers (4) erfaßt und an die Steuereinrichtung (36) übermittelt wird.

8. Extrakorporales Therapiegerät nach einem der vorhergehenden Ansprüche, dadurch gekennzeichnet, daß zur Erfassung der Schwenkstellung des Wandlers (4) ein Wegaufnehmer (37) am Wandler (4) vorgesehen ist.

9. Extrakorporales Therapiegerät nach einem der vorhergehenden Ansprüche, dadurch gekennzeichnet, daß die Kupplungseinrichtung (14-21), mit der das Traggestell (8, 13) der Röntgenortungseinrichtung am Wandler (4) festlegbar ist, traggestellseitig nahe der Röntgenquelle (9) und therapiegerätseitig nahe dem Wandler (4) angeordnet ist.

10. Extrakorporales Therapiegerät nach einem der vorhergehenden Ansprüche, dadurch gekennzeichnet, daß der Wandler (4) durch eine Vielzahl von kalottenförmig angeordneten piezoelektrischen Elementen gebildet ist und daß in dieser Wandlerkalotte ein Fenster und ein Röntgentubus für die Röntgenquelle (9) vorgesehen ist.

11. Verfahren zum Einrichten einer externen Röntgenortungseinrichtung an einem Therapiegerät nach einem der vorhergehenden Ansprüche, gekennzeichnet durch folgende Verfahrensschritte:
a) Ankuppeln der Röntgenortungseinrichtung an den Wandler des Therapiegeräts,
b) Schwenken des Wandlers zusammen mit der Röntgenortungseinrichtung in eine Endstellung,
c) Anbringen einer röntgenpositiven Zielmarke für die Röntgenortung im Fokus des Wandlers,
d) Nachführen der elektronischen Zielmarke auf die tatsächlich auf der Bildwiedergabeeinrichtung der Röntgenortungseinrichtung abgebildete Zielmarke und Speicherung dieser Einstellung in der Steuereinrichtung,
e) Schwenken des Wandlers zusammen mit der Röntgenortungseinrichtung in die andere Endstellung,
f) Nachführen der elektronischen Zielmarke auf die tatsächlich auf der Bildwiedergabeeinrichtung der Röntgenortungseinrichtung abgebildete Zielmarke und Speicherung dieser Einstellung in der Steuereinrichtung und
g) Entfernen der Zielmarke aus dem Fokus.

## Claims

1. Extracorporeal therapy apparatus(1) with an electroacoustic transducer (4) for the generation of focused sound waves, with a patient support (5) displaceable with respect to the transducer (4) for the purpose of positioning a patient and with an X-ray locating device (2), consisting essentially of an X-ray source (9), an X-ray image receiving device (10) and a support frame (8) connecting the latter, wherein the transducer (4) together with the X-ray locating device (2) with reference to the patient support (5) is arranged pivotably about the focus (6) of the transducer in such a manner that the X-ray locating device (2) follows the pivotal movement of the transducer (4), characterised in that the X-ray locating device (2) is constructed as a separate device and is connected in detachable manner through a coupling device to the therapy device and that an X-ray positive tracking mark (22) which can be introduced into the focus (6) of the transducer (4) is provided for the X-ray locating device (2) on the therapy apparatus (1).

2. Extracorporeal therapy device according to claim 1, characterised in that the tracking mark (22) is seated at the end of a retractable and expandable telescope device (29) arranged on the transducer side.

3. Extracorporeal therapy device according to claim 1, characterised in that the tracking mark can be attached to a coupling diaphragm (30) of the transducer (4) which can be placed on the patient.

4. Extracorporeal therapy device according to claim 3, characterised in that on or within the transducer (4) two light sources (41), preferably lasers, are arranged, the beams (42) of which intersect one another in the focus (6) of the transducer (4).

5. Extracorporeal therapy device according to one of the preceding claims, characterised in that an ultrasonic scanner (23) is arranged on the transducer (4) and that the tracking mark (22) is arranged close to the end of the ultrasonic scanner (23) on the patient side such that it can be swung out.

6. Extracorporeal therapy device according to one of the preceding claims, characterised in that the X-ray image receiving device (10) comprises an electronic image reproduction device (33) on which an electronic tracking mark (E) can be represented and that the electronic tracking mark (E) can track on to the tracking mark (M) actually depicted.

7. Extracorporeal therapy device according to one of the preceding claims, characterised in that associated with the X-ray locating device (2) is an electronic control device (36) which tracks the electronic tracking mark (E) during the joint pivoting of the transducer (4) and X-ray locating device (2) on a previously determined course (K), for which purpose the pivot position of the transducer (4) at any time is captured and passed on to the control device (36).

8. Extracorporeal therapy device according to one of the preceding claims, characterised in that a displacement pickup (37) is provided on the transducer (4) for capturing the pivot position of the transducer (4).

9. Extracorporeal therapy device according to one of the preceding claims, characterised in that the coupling device (14-21), with which the support frame (8, 13) of the X-ray locating device can be fixed on the transducer (4), is arranged on the support frame side close to the X-ray source (9) and on the therapy device side close to the transducer (4).

10. Extracorporeal therapy device according to one of the preceding claims, characterised in that the transducer (4) is formed by a multiplicity of piezoelectric elements arranged in the shape of a calotte and that a window and an X-ray tube for the X-ray source (9) is provided in this transducer calotte.

11. Method for the setting up of an external X-ray locating device on a therapy device according to one of the preceding claims, characterised by the following procedural steps:
a) coupling the X-ray locating device to the transducer of the therapy device,
b) pivoting the transducer together with the X-ray locating device into an end position,
c) attaching an X-ray positive tracking mark for the X-ray locating in the focus of the transducer,
d) tracking the electronic tracking mark on to the tracking mark actually depicted in the image reproduction device of the X-ray locating device and storing this setting in the control device,
e) pivoting the transducer together with the X-ray locating device into the other end position,
f) tracking the electronic tracking mark on to the tracking mark actually depicted in the image reproduction device of the X-ray locating device and storing this setting in the control device, and
g) removing the tracking mark from the focus.

## Revendications

1. Appareil extra-corporel de thérapie (1) comportant un transducteur électroacoustique (4) servant à produire des ondes acoustiques focalisées, une couchette pour patient (5) déplaçable par rapport au transducteur (4) pour positionner un patient, et un dispositif de localisation radiographique (2) - constitué essentiellement par une source de rayons X (9), un dispositif (10) de réception de radiographies et un châssis de support (8) relié à ce dispositif, le transducteur (4) étant disposé de manière à pouvoir basculer, conjointement avec le dispositif de localisation radiographique (2), autour du foyer (6) du transducteur par rapport à la couchette pour patient (5) de telle sorte que le dispositif de localisation radiographique (2) suit le mouvement de basculement du transducteur (4), caractérisé en ce que le dispositif de localisation radiographique (2) est agencé sous la forme d'un appareil séparé et est relié de façon amovible à l'appareil de thérapie par l'intermédiaire d'un dispositif d'accouplement, et qu'il est prévu, sur l'appareil de thérapie (1), une marque de visée (22) positive pour les rayons X, qui peut être placée au foyer (6) du transducteur (4), pour le dispositif de localisation radiographique (2).

2. Appareil extra-corporel de thérapie selon la revendication 1, caractérisé en ce que la marque de visée (22) est disposée à l'extrémité d'un dispositif télescopique rétractable et déployable (29), disposé sur un côté du transducteur.

3. Appareil extra-corporel de thérapie selon la revendication 1, caractérisé en ce que la marque de visée peut être apposée sur une membrane de couplage (30) du transducteur (4) qui peut être appliquée contre le patient.

4. Appareil extra-corporel de thérapie selon la revendication 3, caractérisé en ce que sur ou à l'intérieur du transducteur (4) sont disposées deux sources de lumière (41), de préférence des lasers, dont les faisceaux (42) se recoupent au foyer (6) du transducteur (4).

5. Appareil extra-corporel de thérapie selon l'une des revendications précédentes, caractérisé en ce qu'un scanner à ultrasons (23) est disposé sur le transducteur (4) et que la marque de visée (22) est disposée, de manière à pouvoir basculer, à proximité de l'extrémité du scanner à ultrasons (23), située du côté du patient.

6. Appareil extra-corporel de thérapie selon l'une des revendications précédentes, caractérisé en ce que le dispositif (10) de réception de radiographies possède un dispositif électronique de reproduction d'images (33), sur lequel peut être représenté une marque électronique de visée (E), et que la marque électronique de visée (E) peut être asservie à la marque de visée (M) dont l'image est effectivement formée.

7. Appareil extra-corporel de thérapie selon l'une des revendications précédentes, caractérisé en ce qu'au dispositif de localisation radiographique (2) est associé un dispositif de commande électronique (36), qui asservit la marque de visée électronique (E) sur une piste (K) déterminée au préalable, lors du basculement commun du transducteur (4) et du dispositif de localisation radiographique (2), la position basculée respective du transducteur (4) étant à cet effet détectée et transmise au dispositif de commande (36).

8. Appareil extra-corporel de thérapie selon l'une des revendications précédentes, caractérisé en ce que pour détecter la position basculée du transducteur (4) il est prévu, sur le transducteur (4), un capteur de déplacement (37).

9. Appareil extra-corporel de thérapie selon l'une des revendications précédentes, caractérisé en ce que le dispositif d'accouplement (14-21), à l'aide duquel le châssis de support (8,13) du dispositif de localisation radiographique peut être fixé sur le transducteur (4), est disposé, du côté du châssis de support, à proximité de la source de rayons X (9) et, du côté de l'appareil de thérapie, à proximité du transducteur (4).

10. Appareil extra-corporel de thérapie selon l'une des revendications précédentes, caractérisé en ce que le transducteur (4) est formé par une multiplicité d'éléments piézoélectriques disposés sous la forme d'une calotte et qu'une fenêtre et un tube radiographique pour la source de rayons X (9) sont prévus dans cette calotte du transducteur.

11. Procédé pour diriger un dispositif externe de localisation radiographique sur un appareil de thérapie selon l'une des revendications précédentes, caractérisé par les étapes opératoires suivantes :
a) accouplement du dispositif de localisation radiographique au transducteur de l'appareil de thérapie,
b) basculement du transducteur, conjointement avec le dispositif de localisation radiographique, dans une position d'extrémité,
c) mise en place d'une marque de visée, positive pour les rayons X, pour la localisation radiographique au foyer du transducteur,
d) asservissement de la marque de visée électronique sur la marque de visée dont l'image effectivement est formée sur le dispositif de reproduction d'image du dispositif de localisation radiographique, et mémorisation de ce réglage dans le dispositif de commande,
e) basculement du transducteur, conjointement avec le dispositif de localisation radiographique, dans l'autre position d'extrémité,
f) asservissement de la marque de visée électronique sur la marque de visée, dont l'image est effectivement formée sur le dispositif de reproduction d'image du dispositif de localisation radiographique, et mémorisation de ce réglage dans l'unité de commande, et
g) retrait de la marque de visée hors du foyer.
